# EUROPÄISCHE PATENTANMELDUNG

(11) **EP 0 729 785 A1**
(43) Veröffentlichungstag der Anmeldung: **04.09.1996**
(21) Anmeldenummer: 96102407.2
(22) Anmeldetag: 17.02.1996
(51) Int. Cl.: B01J 23/46, C07C 213/02

(54) **Katalysatoren für die Amierung von Alkoholen, Ketonen und Aldehyden**

(30) Priorität: 28.02.1995 DE 19507007
(71) Anmelder: BASF AKTIENGESELLSCHAFT, 67056 Ludwigshafen (DE)
(72) Erfinder: Melder, Johann-Peter, Dr., 67141 Neuhofen (DE); Wulff-Döring, Joachim, Dr., 67227 Frankenthal (DE); Gutschoven, Frank, 67063 Ludwigshafen (DE); Witzel, Tom, Dr., 67069 Ludwigshafen (DE); Harder, Wolfgang, Dr., 69469 Weinheim (DE)

(57) **Zusammenfassung**

Katalysatoren für die Aminierung von Alkoholen, Ketonen oder Aldehyden bestehend aus 0,001 bis 25 Gew.-% Ruthenium und 0 bis 5 Gew.-% Promotoren aus der Gruppe Eisen, Rhodium, Palladium, Platin, Iridium, Osmium, Kupfer, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder deren Gemische auf porösen Oxiden sowie ein Verfahren zur Herstellung von Aminen.

## Beschreibung

Die vorliegende Erfindung betrifft neue Katalysatoren, bestehend aus Ruthenium und gegebenenfalls von Promotoren auf porösen Oxiden, für die Umwandlung von Alkoholen, Ketonen und Aldehyden zu Aminen, speziell für die selektive Aminierung von Monoethanolamin (MEA) zu Ethylendiamin (EDA).

Aus der DE-A-40 10 227 sind Ruthenium-Trägerkatalysatoren zur Hydrierung von Iminen zu Aminen bekannt.

Aus der EP-A-146 508 sind Nickel- bzw. Cobaltkatalysatoren auf porösen Metalloxidträgern für Hydrier-Dehydrierreaktionen bekannt, die mit Rutheniumchlorid dotiert sind. Bei diesen Katalysatoren werden die Aktivkomponenten in einer aufwendigen Prozedur nacheinander aufgetränkt, wobei zwischen den einzelnen Tränkschritten noch zusätzlich getrocknet und gegebenenfalls auch calciniert werden muß.

In der EP-A-254 335 wurde auf die Rutheniumaufbringung als Chlorid verzichtet, indem man das Trägermaterial vorab mit 18 %iger HCl behandelt. Da die meisten Metalloxide im stark Sauren nicht stabil sind, leidet die mechanische Stabilität, wodurch sich die Standzeit im Betrieb aufgrund von Katalysatorzerfall verringert.

Der vorliegenden Erfindung lag daher die Aufgabe zugrunde, den zuvor genannten Nachteilen abzuhelfen.

Demgemäß wurden neue und verbesserte Katalysatoren für die Aminierung von Alkoholen, Ketonen oder Aldehyden bestehend aus 0,001 bis 25 Gew.-% Ruthenium und 0 bis 5 Gew.-% Promotoren aus der Gruppe Eisen, Rhodium, Palladium, Platin, Iridium, Osmium, Kupfer, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder deren Gemische auf porösen Oxiden gefunden sowie ein Verfahren zur Herstellung von Aminen.

Die erfindungsgemäßen Katalysatoren lassen sich wie folgt herstellen:

Die Ruthenium-Katalysatoren können durch Tränken des Trägers mit einer Lösung von Ruthenium und gegebenenfalls durch Zusatz von Promotoren hergestellt werden. Dabei kann die Tränkung entweder gleichzeitig oder nacheinander durchgeführt werden. Möglich ist auch das Aufsprühen der gelösten Aktivkomponente und gegebenenfalls von Promotoren auf den Träger. Die Aktivkomponente kann auch durch gemeinsames Verkneten oder Fällung mit dem Trägermaterial vereinigt werden. Bei dieser Herstellungsmethode wird die Katalysatormasse in der Regel anschließend verformt.

Die Tränklösung kann aus den Salzen des Rutheniums, z.B. der Nitrate, Chloride, Formiate, Oxalate oder Ammoniakate, bevorzugt der Chloride und Nitrate, besonders bevorzugt der Chloride hergestellt werden.

Als porösen Oxide eignen sich z.B. Aluminiumoxid, Siliciumdioxid, Alumosilikate, Titandioxid, Zirkoniumoxid und Magnesiumoxid, jeweils in allen Modifikationen, bevorzugt Aluminiumoxid, Siliciumdioxid, Alumosilikate, besonders bevorzugt Aluminiumoxid. Der Einsatz anderer Träger als Aluminiumoxid kann besonders dann vorteilhaft sein, wenn neben primären Aminen höhere Anteile sekundärer Amine (bei der Monoethanolamin-Aminierung z.B. C₄-Produkten wie Aminoethylethanolamin oder Diethylentriamin) erwünscht sind.

Der Rutheniumgehalt des Katalysators, bezogen auf das Trägermaterial, liegt in der Regel bei 0,001 bis 25 Gew.-%, bevorzugt 0,1 bis 20 Gew.-%, besonders bevorzugt 1 bis 15 Gew.-%. Man kann die Katalysatoren beispielsweise in Form von Formkörpern, z.B. Strängen, Tabletten oder als Pulver, je nach vorgesehenem Verwendungszweck, einsetzen.

Die Selektivität des Katalysators kann zusätzlich durch Dotierung mit den Promotoren Eisen, Rhodium, Palladium, Platin, Iridium, Osmium, Kupfer, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder deren Gemische gesteuert werden.

Der Promotorgehalt des Katalysators, bezogen auf das Trägermaterial, liegt in der Regel bei 0 bis 5 Gew.-%, bevorzugt 0,001 bis 5 Gew.-%, besonders bevorzugt 0,01 bis 3 Gew.-%.

Vor dem Einsatz als Katalysator müssen diese zunächst bei 80 bis 150°C, bevorzugt 80 bis 120°C getrocknet werden. Anschließend werden die Katalysatoren zwischen 150 und 500°C calciniert. Die Reduktion der abgekühlten Probe findet bei Temperaturen zwischen 150 und 500°C im Wasserstoff- bzw. Wasserstoff/Inertgas-Strom statt. Diese Aktivierung kann direkt im Synthese-Reaktordurchgeführt werden. Falls die Reduktion in einem separaten Reaktor durchgeführt wird, müssen die Katalysatoren vor dem Ausbau bei 30°C mit sauerstoffhaltigen Gasgemischen oberflächlich passiviert werden. Die passivierten Katalysatoren werden in diesem Fall im Synthese-Reaktor vor dem Einsatz in einem Wasserstoffstrom bei 180°C aktiviert.

Die so erhaltenen Katalysatoren können neben der Aminierung von Alkoholen auch vorteilhaft bei der reduktiven Aminierung von Ketonen und Aldehyden eingesetzt werden, speziell bei der Umsetzung von Monoethanolamin mit Ammoniak zu Ethylendiamin.

Die neuen Katalysatoren sind stabil und favorisieren die Bildung primärer Amine. Diese Katalysatoren zeigen überraschenderweise bei gleicher Aktivität wie die Ru-dotierten Nickel- und Cobalt-Katalysatoren eine höhere Selektivität und zusätzlich eine deutlich höhere Standzeit. Auf die klassischen Aminierungsmetalle Cobalt und Nickel kann vollständig verzichtet werden. Neben der Einsparung dieser Metalle vereinfacht sich dadurch gegenüber dem Stand der Technik auch die Herstellung der Katalysatoren.

Die Aminierung kann diskontinuierlich oder bevorzugt kontinuierlich in einem Rohrreaktor in Riesel- oder Sumpffahrweise durchgeführt werden. Man arbeitet bei der Aminierung von Alkoholen bei Temperaturen von 120 bis 250°C, bevorzugt von 150 bis 190°C, und bei Drücken von 150 bis 300 bar, bevorzugt bei 180 bis 220 bar.

Als Reagenz und Lösungsmittel wird Ammoniak eingesetzt. Der Ammoniaküberschuß liegt zwischen 1 und 20 mol/mol Alkohol, bevorzugt zwischen 6 und 12 mol/mol Alkohol. Die Alkohol-Belastung liegt je nach Aktivität des Katalysators zwischen 0,1 und 1,0 kg/l*h. Durch Veränderung der Verweilzeit kann der Umsatz und damit die Selektivität gezielt eingestellt werden.

Die Substituenten R¹, R², R³, R⁴, R⁵ und die Indizes l, m und n in den Verbindungen I, II und III haben unabhängig voneinander folgende Bedeutungen:
R¹, R², R³, R⁴, R⁵, R⁶
   - Wasserstoff,
R¹, R²
   - C₁- bis C₂₀₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀- bis C₂₀₀-Alkyl wie Polybutyl, Polyisobutyl, Polypropyl, Polyisopropyl und Polyethyl, besonders bevorzugt Polybutyl und Polyisobutyl,
   - R¹ und R² gemeinsam eine -(CH₂)ₗ-X-(CH₂)ₘ-Gruppe,
R¹, R², R³, R⁶
   - C₃- bis C₁₂-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R¹
   - C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Di-methylpropyl, n-Hexyl, iso-Hexyl, sec.-Hexyl, n-Heptyl, iso-Heptyl, n-Octyl, iso-Octyl, besonders bevorzugt C₁-bis C₄-Alkyl wie Methyl, Ethyl, n-Propyl, iso-Propyl, n-Butyl, iso-Butyl, sec.-Butyl und tert.-Butyl,
R³, R⁶
   - C₁- bis C₂₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methyl-ethyl,
   - durch Amino und/oder Alkylamino und/oder Dialkylamino und/oder Hydroxy substituiertes C₁- bis C₂₀-Alkyl, bevorzugt durch Amino und/oder Alkylamino und/oder Dialkylamino und/oder Hydroxy substituiertes C₁- bis C₈-Alkyl, besonders bevorzugt durch Amino und/oder Alkylamino und/oder Dialkylamino und/oder Hydroxy substituiertes C₁- bis C₄-Alkyl wie N-(Hydroxyethyl)aminoethyl und N-(Aminoethyl)aminoethyl,
   - C₂- bis C₃₀-Alkoxyalkyl, bevorzugt C₂- bis C₂₀-Alkoxyalkyl, besonders bevorzugt C₂- bis C₈-Alkoxyalkyl wie Methoxymethyl, Ethoxymethyl, n- Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxy-ethyl, besonders bevorzugt C₂- bis C₄-Alkoxyalkyl wie Methoxymethyl, Ethoxy- methyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
   - R⁴-(OCHR⁵CH₂)ₙ,
R⁴
   - C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
R⁵
   - Methyl,
X
   - Sauerstoff,
   - N-R⁵,
l
   - eine ganze Zahl von 2 bis 4 wie 2, 3 und 4, bevorzugt 2 und 3, besonders bevorzugt 2,
m
   - eine ganze Zahl von 1 bis 4 wie 1, 2, 3 und 4, bevorzugt 2, 3 und 4, besonders bevorzugt 2 und 3,
n
   - eine ganze Zahl von 2 bis 10, bevorzugt eine ganze Zahl von 2 bis 8 wie 2, 3, 4, 5, 6, 7 oder 8, besonders bevorzugt eine ganze Zahl von 2 bis 6 wie 2, 3, 4, 5 oder 6,
Z
   - CHR⁶-OH,
   - 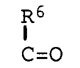 oder
   - CHO.

Die erfindungsgemäß erhältlichen Amine eignen sich u.a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

### Beispiele

### Beispiel 1

175 g 4 mm-Al₂O₃-Stränge (D10-10, BASF) wurden mit 114 ml einer wäßrigen 9,2 %-igen RuCl₃-Lösung, die 3,75 g Ruthenium enthielt, unter mehrmaligem, gutem Durchmischen bei Raumtemperatur 2 h stehengelassen. Anschließend wurde der Katalysatorvorläufer 16 h bei 120°C getrocknet und 4 h bei 400°C calciniert. Nach dem Abkühlen wurden die Stränge in eine Reduktionsapparatur eingebaut und 2 h mit 20 l N₂/h gespült. Danach wurde mit 2°C/min und 20 l H₂/h auf 300°C aufgeheizt und 20 h bei dieser Temperatur gehalten. Nach Abkühlen im Stickstoffstrom wurde der Katalysator mit einem Luft-Stickstoff-Gemisch passiviert, wobei die Temperaturerhöhung max. 20°C betragen darf. Der so hergestellte Katalysator enthielt 2% Ruthenium auf Aluminiumoxid. Die Ergebnisse bei der Aminierung von Monoethanolamin sind in Tabelle 1 zusammengestellt. Der Katalysator war nach einer Betriebszeit von 250 h noch vollständig erhalten.

### Beispiel 2

Dieser Katalysator wurde analog Beispiel 1 hergestellt, nur daß eine wäßrige 4,04 %-ige RuCl₃-Lösung verwendet wurde. Der Katalysator enthielt 1% Ruthenium auf Aluminiumoxid.

### Beispiel 3

Dieser Katalysator wurde analog Beispiel 1 hergestellt, nur daß eine eine wäßrige 22,7 %-ige RuCl₃-Lösung verwendet wurde. Der Katalysator enthielt 5% Ruthenium auf Aluminiumoxid.

### Beispiel 4

Dieser Katalysator wurde analog Beispiel 1 hergestellt, nur daß Siliciumdioxid (D11-10, BASF) als Träger verwendet wurde. Der Katalysator enthielt 2% Ruthenium auf Siliciumdioxid.

### Beispiel 5

Dieser Katalysator wurde analog Beispiel 1 hergestellt, nur daß Titandioxid (DT 51, Rhone Poulenc) als Träger verwendet wurde. Der Katalysator enthielt 2% Ruthenium auf Titandioxid.

### Beispiel 6

Dieser Katalysator wurde analog Beispiel 1 hergestellt, nur daß Zirkoniumdioxid (D12-12, BASF) als Träger verwendet wurde. Der Katalysator enthielt 2% Ruthenium auf Zirkoniumdioxid.

### Beispiel 7

Dieser Katalysator wurde analog Beispiel 1 hergestellt, nur daß eine Lösung aus 9,0 Gew.-% RuCl₃ und 19,5 Gew.-% Cu(NO₃)₂ verwendet wurde. Der Katalysator enthielt 2% Ruthenium und 4% Kupfer auf Aluminiumoxid.

### Vergleichsbeispiel A

Der Katalysator wurde nach EP-A-254 335, Beispiel 1 hergestellt. Der so erhaltene Katalysator enthielt 10% Nickel und 0,5% Ruthenium auf Aluminiumoxid (D 10-10, BASF). Die Ergebnisse bei der Aminierung von Monoethanolamin sind in Tabelle 1 zusammengestellt. Der Katalysator war nach einer Betriebszeit von 48 h vollständig zerfallen.

### Vergleichsbeispiel B

Der Katalysator wurde nach EP-A-254 335, Beispiel 13 hergestellt. Der so erhaltene Katalysator enthielt 10% Cobalt und 0,5% Ruthenium auf Aluminiumoxid (D 10-10, BASF). Ergebnisse bei der Aminierung von Monoethanolamin sind in Tabelle 1 zusammengestellt. Der Katalysator war nach einer Betriebszeit von 48 h vollständig zerfallen.

Bei der Umsetzung von Monoethanolamin mit Ammoniak lieferten die neuen Katalysatoren 1 bis 7 und die Vergleichsbeispiele A und B folgende Ergebnisse:

### Abkürzungen:

EDA = Ethylendiamin
AEEA = Aminoethylethanolamin
DETA = Diethylentriamin

### Katalysatortest

Ein 100 ml Rohrreaktor mit einer Länge von 55 cm und einem Innendurchmesser von 1,5 cm wurde mit 50 g passiviertem Katalysator befüllt und der Katalysator bei 180°C zunächst mit einem Gemisch aus 20 Vol.-% Wasserstoff / 80 Vol.-% Stickstoff und anschließend mit 100 Vol.-% Wasserstoff aktiviert. Nach Einstellung der Reaktionstemperatur je nach Aktivität des Katalysators auf 165 bis 220°C wurde der Reaktor mit 10 bis 30 g/h Monoethanolamin, 20 bis 70 g/h Ammoniak und 3 bis 10 Nl/h Wasserstoff beschickt und durch gaschromatographische Analyse des Austrags der Umsatz und die Selektivität bezüglich der Komponenten Ethylendiamin, Aminoethylethanolamin, Diethylentriamin und Piperazin bestimmt.

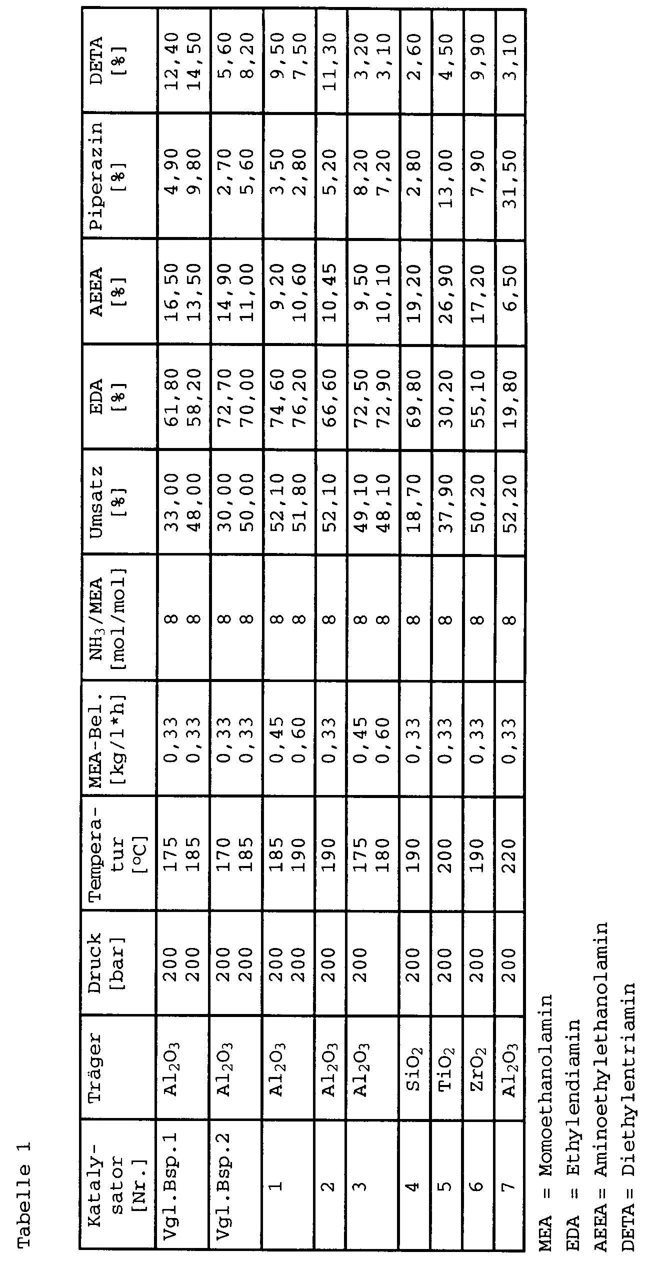

## Patentansprüche

1. Katalysatoren für die Aminierung von Alkoholen, Ketonen oder Aldehyden bestehend aus 0,001 bis 25 Gew.-% Ruthenium und 0 bis 5 Gew.-% Promotoren aus der Gruppe Eisen, Rhodium, Palladium, Platin, Iridium, Osmium, Kupfer, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder deren Gemische auf porösen Oxiden.

2. Katalysatoren für die Aminierung von Alkoholen, Ketonen oder Aldehyden nach Anspruch 1 bestehend aus 0,1 bis 20 Gew.-% Ruthenium und 0 bis 5 Gew.-% Promotoren auf porösen Oxiden.

3. Katalysatoren für die Aminierung von Alkoholen, Ketonen oder Aldehyden nach Anspruch 1 bestehend aus 1 bis 15 Gew.-% Ruthenium und 0 bis 5 Gew.-% Promotoren auf porösen Oxiden.

4. Katalysatoren für die Aminierung von Alkoholen, Ketonen oder Aldehyden nach Anspruch 1, dadurch gekennzeichnet, daß man als Promotoren Rhodium, Palladium, Platin, Kupfer, Silber oder deren Gemische einsetzt.

5. Katalysatoren für die Aminierung von Alkoholen, Ketonen oder Aldehyden nach Anspruch 1, dadurch gekennzeichnet, daß man als Promotor Kupfer einsetzt.

6. Katalysatoren für die Aminierung von Alkoholen, Ketonen oder Aldehyden nach Anspruch 1, dadurch gekennzeichnet, daß man als poröse Oxide Aluminiumoxid, Siliciumdioxid, Alumosilikate, Titandioxid, Zirkoniumoxid, Magnesiumoxid oder deren Gemische einsetzt.

7. Verfahren zur Herstellung von Aminen der allgemeinen Formel I 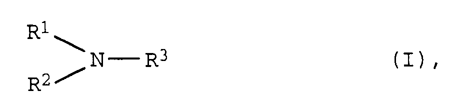 in der
R¹ und R² Wasserstoff, C₁- bis C₂₀₀-Alkyl, C₃- bis C₁₂-Cycloalkyl, durch Amino, Alkylamino, Dialkyl-amino und/oder Hydroxy substituiertes C₁- bis C₂₀-Alkyl, C₂-bis C₃₀-Alkoxyalkyl, R⁴-(OCHR⁵CH₂)ₙ, Aryl, C₇- bis C₂₀-Aralkyl und C₇- bis C₂₀-Alkylaryl oder gemeinsam (CH₂)ₗ-X-(CH₂)ₘ,
R³, R⁶ Wasserstoff, C₁- bis C₂₀-Alkyl, C₃- bis C₁₂-Cycloalkyl, Aryl, C₇- bis C₂₀-Aralkyl und C₇- bis C₂₀-Alkylaryl,
R⁴ Wasserstoff, C₁- bis C₄-Alkyl,
R⁵ Wasserstoff oder Methyl,
X Sauerstoff oder N-R⁵,
n eine ganze Zahl von 2 bis 30,
l eine ganze Zahl von 2 bis 4,
m eine ganze Zahl von 1 bis 4
bedeuten, aus primären oder sekundären Alkoholen, Ketonen oder Aldehyden der allgemeinen Formel II
R³ - Z (II),
in der Z für CHR⁶-OH, 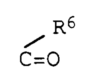 oder CHO und R⁶ für Wasserstoff, C₁- bis C₂₀-Alkyl, C₃-bis C₁₂-Cycloalkyl, Aryl, C₇- bis C₂₀-Aralkyl und C₇- bis C₂₀-Alkylaryl steht, und Stickstoffverbindungen der allgemeinen Formel III 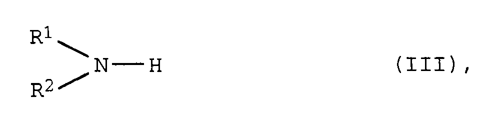 in der R¹, R² die oben genannten Bedeutungen haben und R¹ und R² zusätzlich für Wasserstoff steht, bei Temperaturen von 80 bis 250°C und Drücken von 1 bis 400 bar in Gegenwart eines Katalysators, dadurch gekennzeichnet, daß man Katalysatoren bestehend aus 0,001 bis 25 Gew.-% Ruthenium und 0 bis 5 Gew.-% Promotoren aus der Gruppe Eisen, Rhodium, Palladium, Platin, Iridium, Osmium, Silber, Gold, Chrom, Molybdän,Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder deren Gemische auf porösen Oxiden, einsetzt.

8. Verfahren zur Herstellung von Aminen nach Anspruch 7, dadurch gekennzeichnet, daß man die Umsetzung bei Temperaturen von 130 bis 230°C durchführt.

9. Verfahren zur Herstellung von Aminen nach Anspruch 7, dadurch gekennzeichnet, daß man Monoethanolamin mit Ammoniak in Gegenwart eines Katalysators durchführt, der aus 0,001 bis 25 Gew.-% Ruthenium und 0 bis 5 Gew.-% Promotoren aus der Gruppe Rhodium, Palladium, Platin, Kupfer, Silber oder deren Gemische auf porösen Oxiden besteht.

10. Verfahren zur Herstellung von Aminen nach Anspruch 7, dadurch gekennzeichnet, daß man Monoethanolamin mit Ammoniak in Gegenwart eines Katalysators durchführt, der aus 0,001 bis 25 Gew.-% Ruthenium und 0 bis 5 Gew.-% Kupfer auf porösen Oxiden besteht.
